# EUROPEAN PATENT APPLICATION

(11) **EP 4 276 462 A1**
(43) Date of publication of application: **15.11.2023**
(21) Application number: 22173239.9
(22) Date of filing: 13.05.2022
(51) Int. Cl.: G01N 33/50

(54) **POLYMER DEVICE FOR IN-VITRO DRUG EVALUATION**

(71) Applicant: Robert Bosch Gesellschaft für medizinische Forschung mbH, 70376 Stuttgart (DE)
(72) Inventor: Kemas, Aurino, Stockholm (SE); Zandi Shafagh, Reza, 18347 Täby, Stockholm (SE); Lauschke, Volker, 11544 Stockholm (SE)
(74) Representative: Bee, Joachim

(57) **Abstract**

The present invention provides a polymer device for in-vitro drug evaluation, characterized in that the device is comprised of a main body comprising an thiol-ene based polymer. In addition, the use of a polymer device comprising an thiol-ene based polymer main body for in-vitro drug evaluation and a method for assaying the toxicity and/or efficacy of at least one drug on a cell culture are provided.

## Description

The invention concerns a polymer device for in-vitro drug evaluation. Particularly, the invention pertains to a polymer device for in-vitro drug evaluation, wherein the polymer device is comprised of a main body comprising an thiol-ene based polymer.

### State of the art

With the technical breakthroughs in scalable and inexpensive production of polymers, they have gradually replaced glas as material for cell culture. At present, most cell culture wares and disposables in laboratories are made of plastics.

However, using polymers is not without drawbacks. This is evident in the case of polydimethylsiloxane (PDMS), which is currently the primary material of choice in the pharmaceutical industry and academia for drug testing, especially with their ease of prototyping, gas permeability, and optical transparency (G. M. Whitesides, Nature. 442 (2006), pp. 368-373).

PDMS offers, however, also disadvantages including the ab- and/or adsorption of small molecules to be tested (M. W. Toepke, D. J. Beebe, Lab Chip. 6 (2006), pp. 1484-1486, B. J. van Meer et al., Biochem. Biophys. Res. Commun. 482, 323-328 (2017)). The increased use of PDMS is further paralleled by gradual adoption of organotypic three-dimensional (3D) perfusable cell culture systems to improve translatability of in-vitro testing in drug development pipeline.

Several efforts have been done to address such difficulties relating to drug ab- and/or adsorption on polymer devices. One approach is by surface activation through oxygen-plasma (David C. Duffy et al., Anal. Chem. 70, 4974-4984 (1998)) or UV-ozone (K. Efimenko et al., J. Colloid Interface Sci. 254, 306-315 (2002)) treatments. However, such treatment is transient. Water contact angle measurement of PDMS treated with oxygen-plasma, for instance, show that oxidation of the polymer renders it more hydrophilic, but after 1 week the contact angle values return almost completely to the initial value (P. M. Van Midwoud et al., Anal. Chem. 84, 3938-3944 (2012)). UV-ozone treatment exhibits the disadvantage that toxic compounds or side group moieties may be produced, such as peroxide, which is formed during the oxidation of a poly(methyl methacrylate) (PMMA) surface (A. Welle, E. Gottwald, Applications. Biomed. Microdevices. 4 (2002)).

### Disclosure of the invention

The invention seeks to overcome the before mentioned problems associated with commonly employed polymer devices, particularly, PDMS .

In particular, the present invention provides a polymer device for in-vitro drug evaluation, characterized in that the device is comprised of a main body comprising an thiol-ene based polymer. Preferably, the thiol-ene based polymer is an off-stoichiometric thiole-ene (OSTE) polymer.

The use of a polymer device comprising an thiol-ene based polymer main body for in-vitro drug evaluation is provided. Preferably, the thiol-ene based polymer main body is an OSTE polymer main body.

In addition, a method for assaying the toxicity and/or efficacy of at least one drug on a cell culture, the method comprising the steps of a. providing a polymer device comprised of a main body comprising an thiol-ene based polymer; b. seeding of a cell culture on the polymer device; c. contacting, over a certain time period, the cell culture on the polymer device with a certain amount of the at least one drug to be assayed; and d. assaying characteristics of the cell culture after the time period of contacting with the at least one drug and comparing the assayed characteristics to the characteristics of the cell culture prior to the time period of contacting with the at least one drug, wherein the characteristics allow conclusions on the toxicity and/or the efficacy of the at least one drug on the cell culture. Preferably, the thiol-ene based polymer main body is an OSTE polymer main body.

### Adantages of the invention

In course of the studies leading to the present invention, the inventors surprsingly have found that thiol-ene based polymers are particularly suitable for the in-vitro drug evaluation using viable cell cultures, particularly organotypic three-dimensional (3D) perfusable cell cultures. The thiol-ene network in thiol-ene based polymer materials inter alia offers good polymerization control, a uniform and high degree of crosslink density with high optical clarity and reduced polymerization shrinkage stress due to delayed gelation.

In particular, it has been found that sorption of a wide variety of drugs, particularly in contrast to PDMS, is negligible in thiol-ene based polymers. Thiol-ene based polymer material has been proven a material that overcomes the problems asociated with other materials, such as PDMS, permitting a reliable testing of drugs of diffrerent nature, such as small molecule compounds, antibodies, antiviral compounds, antibiotics, and bioactive compounds, and different properties, such as polarity and hydrophobicity. In addition, providing a coating to a thiol-ene based polymer may further improve reliable drug testing.

This is particular surprising in view of the fact that off-stoichiometric thiol-enes encompass non-reacted educts, which include typically free thiol groups and/or allyl groups, that are evenly distributed throughout the polymer device and which may interact with the cell cultures, drug, or any other material contacting the polymer device.

### Embodiments of the invention

The term "sorption" as used herein pertains to a combination of absorption and adsorption (with their chemical meaning). In chemistry, absorption is a physical or chemical phenomenon or a process in which atoms, molecules or ions enter some bulk phase - liquid or solid material. Preferably, the solid material is the polymer device. In contrast thereto, in chemistry, adsorption is the adhesion of atoms, ions or molecules from a gas, liquid or dissolved solid to a surface. Preferably, a surface of the polymer device. Preferably, sorption of a drug is the fraction of free drug in the polymer device after a certain incubation time.

A thiol-ene-based polymer comprises blends of thiol containing compounds and allyl containing compounds that can be mixed stoichiometrically or non-stochiometrically (i.e. off-stoichiometrically) and polymerize in a step-growth polymerization process. Said compounds encompass usually two to six thiol groups and two to six allyl groups, respectively. Suitable compounds as well as the prepration of thiol-ene based polymers is known in the art. Preferred thiol-ene based polymers are off-stoichiometric thiole-ene (OSTE) polymers. Whereas no reactive group remains in the bulk and on the surface after polymerization of stoichiometric mixtures, reactive thiol or ene groups remain in the bulk and on the surface after polymerization of off-stoichiometric mixtures. Off-Stoichiometry is obtained by reacting thiol groups and allyl groups with a ratio different from 1:1. A preferred ratio of thiol groups to allyl groups in the educts is 2:1 to 1:2 (without the ratio 1:1, preferably without the reatio 1.05:1 to 1:1.05). A preferred OSTE resin is OSTEMER 322, Sweden, which can be used according to the manufacturer's instructions.

According to an embodiment of the invention, the device is selected from a microfluidic chip, an organ-on-a-chip, a cell-culture-device, and a multiwell plate. Said devices are well known in the art. A microfluidic chip comprises a thiol-ene based polymer material having a set of micro-features (chambers, channels, wells, pillars, gratings) formed therein. Said micro-features may be, e.g., milled, photopatterned, e-beam patterned, imprinted, etched and/or molded into the thiol-ene based polymer material. The micro-features forming the microfluidic chip are connected together in order to achieve the desired functions, including one or more of mixing, pumping, sorting and controlling the biochemical environment. An *organ-on-a-chip* is a multi-channel microfluidic cell culture, integrated circuit (chip) that simulates the activities, mechanics and physiological response of an entire organ or an organ system, and may be regarded a type of artificial organ. An organ-on-a-chip may be prepared like a microfluidic chip. A cell-culture-device allows growth and handling of cells in an artificial environmement. A multiwell plate is a flat plate with multiple "wells" used as small test tubes. Preferred multiwell plates encompass, e.g., 6, 12, 24, 48, 96, or more wells. Multiwell plates may be prepared by fabricating a plurality of wells in a rectangular solid, such as a thiol-ene based polymer.

According to an embodiment of the invention, the device is provided with a cell culture seeded on the device and with at least one drug contacting the cell culture. Any type of cell culture can be used, including animal and human cell cultures, such as a 3D cell culture. Preferred cell cultures are human cell cultures, more preferably 3D human cell cultures, such as a primary human hepatocyte (PHH) spheroid culture. Media composition, cultivation and maintaining conditions, etc. are known in the art. The at least one drug is not particuarly limited. Any drug expected to affect the cell culture, e.g. viability of cells, may be employed. Any number of drugs, preferably a plurality of drugs, such as 10³ or more, preferably 10⁴ or more, or 10⁵ or more, may be subject to an assay. The assay is not particularly limited. An example of a suitable test for qualifying or quantifying cell viability is the ATP assay (CellTiter Glo Promega), which may be performed according to the manufacturer's instructions.

According to an embodiment of the invention, the at least one drug is selected from small molecule compounds, antibodies, antiviral compounds, antibiotics, and bioactive compounds. Small molecule compounds include compounds with a molecular weight of 1000 g/mol or less, preferably 100 g/mol to 900 g/mol, such as 200 g/mol to 800 g/mol, 300 g/mol to 700 g/mol, 400 g/mol to 600 g/mol, or 450 g/mol to 550 g/mol. It will be appreciated that a given compound may fall in more than one of the above mentioned categiories, for instance Penicillins may be regarded as a small molecule, antibiotic, and bioactive compound. Examplary drugs include chlorpromazine hydrochloride (CPZ), acetaminophen (APAP), montelukast sodium (MON), and tamoxifen (TAM).

According to an embodiment of the invention, the at least one drug includes a drug having a logP of about 3.0 to about 7.5.Preferred is a logP of about 3.5 to about 7.0, more preferred of logP of CPZ (about 4.54) to logP of TAM (about 6.35). LogP pertains to the n-octanol-water partition coefficient (also denominated n-octanol-water partition ratio or Kow) and is a partition coefficient for the two-phase system consisting of n-octanol and water. LogP values may be derived from literature. Alternatively, in case a literature value is not availabe, logP may be measured or calculated according to procedures known in the art. More preferred are at least 10 drugs, such as at least 100 drugs, or at least 1000 drugs having a logP of about 3.0 to about 7.5, such as a logP of about 3.5 to about 7.0, such as a logP of CPZ (about 4.54) to logP of TAM (about 6.35).

According to an embodiment of the invention, the polymer device comprises a layer coated on the main body. The layer coated on the main body or coating is preferably a biocompatible hydrophilic coating that is provided to the a thiol-ene based polymer by adsorption or a chemical modification of the polymer surfaces via grafting techniques. Suitable grafting techniques for PDMS are disclosed for instance in I. Wong, C.-M. Ho, Microfluid. Nanofluidics 2009 73. 7, 291-306 (2009), CN 105829369 A, WO 2009/008547 A1, WO 00/40627 A1, WO 2020/156907 A1, and WO 2019/025207A1. Another possible coating is Biofloat by faCellitate, Germany, which can be employed according to the manufacturer's instructions.

According to an embodiment of the invention, the in-vitro drug biocompatible hydrophilic coatings evaluation is in-vitro drug toxicity evaluation and/or in-vitro drug efficiacy evaluation.Toxicity is the degree to which a drug can damage an organism. Toxicity preferably refers to the effect on a cell (cytotoxicity) or cell culture or an organ such as the liver (hepatotoxicity). Drug toxicity may involve one or more of dose-dependent reactions, allergic reactions, idiosyncratic reactions, and drug-drug interactions. Toxic effects of drugs may be further classified as pharmacological, pathological, or genotoxic. Drug efficiacy is the response, preferably of a cell culture, achievable from an applied or dosed drug.

According to an embodiment of the invention, the in-vitro drug evaluation is performed with a cell culture seeded on the device and with at least one drug. Cell culture and the at least one drug drug may be as described above. The at least one drug may be added at any time to the cell culture that has been seeded on the device. For instance, the at least one drug drug may be provided to the device bevore seeding the cell culture, concomittantly with seeding the cell culture, or after seeding the cell culture.

The invention is now described and explained in further detail by referring to the following non-limiting examples and figures.

### Description of the figures

Fig. 1 shows that inappropriate choice of polymer substrates may bias drug testing outcomes. In particular, Fig. 1a shows PHH spheroid exposure to different doses of drugs in either PDMS or thiol-ene-based devices, representing the high- and low-sorbing polymer substrates, respectively, Fig. 1b shows brightfield images of representative PHH spheroids exposed with different doses of CPZ, Fig. 1c show toxicity assay result, and
Fig. 2 shows that coatings may improve in some cases the difficulties associated with drug sorption.

### Examples

### 1. Materials and methods

### 1.1 Chemicals

The following chemicals are purchased from Sigma Aldrich (Sweden) and have purity of at least 98% (HPLC grade): chlorpromazine hydrochloride (CPZ), acetaminophen (APAP), montelukast sodium (MON), and tamoxifen (TAM).

### 1.2 Fabrication of polymer devices

Polymer devices are fabricated via molding or CNC micromilling (Minitech Machinery Corp., USA) of cyclic olefin copolymer (COC), poylstyrene (PS), polycabonate (PC), polypropylene (PP), poly(methyl methacrylate) (PMMA) and poly(tetrafluoro ethylene) (PTFE) polymers. The devices are comprised of standard 96 wells with a diameter of 6 mm, depth of 3.2 mm and pitch of 9 mm. For PDMS and OSTE polymers, the casting of polymer precursors is implemented for the replication of the devices. Aluminum molds are micromilled using the same milling machine as previously described. Aluminum molds feature a square cavity with an array of 16 pillars with a diameter of 6 mm, depth of 3.3 mm and pitch of 18 mm. Next, OSTE resin (OSTEMER 322, Sweden) and PDMS polymer precursors (Sylgard 184, Dow Corning, US) are mixed and degassed separately. Next to that, each polymer resin is cast in an aluminum mold. OSTE resin is exposed to collimated UV-light (14 mW cm⁻²) for 120 s and then thermally cured at 70 °C overnight. PDMS is only subject to thermal curing at 70 °C for 1 hour. Subsequent to curing OSTE and PDMS polymers are demolded.

### 1.3 Cell culture and toxicity testing

Cryopreserved PHHs (3 donors in total) are seeded and maintained as previously reported in C. C. Bell et al., OPEN. Nat. Publ. Gr. (2016), doi:10.1038/srep25187. Prior to toxicity screening, the devices are disinfected with 70% ethanol and air-dried for at least 30 min. Warm culture medium, according to C. C. Bell et al., containing appropriate concentrations of the drugs is dispensed into wells in each polymer device. DMSO is used as a vehicle to dissolve the drugs with maximum final concentration of 0.5% as how hepatotoxicity screenings have been routinely performed (S. U. Vorrink et al. ,Toxicol. Sci. 163, 655-665 (2018)). A single PHH spheroid is then loaded into each well and the device is covered immediately. Each device is maintained in 37°C incubator (5% CO₂) in a humidity chamber to minimize evaporation.

Following 24h of drug treatments with MON, TAM, and CPZ (except for APAP where the exposure is prolonged to 48h to observe dose-dependent effects), brightfield images are obtained and cell viability is quantified using ATP assay (CellTiter Glo Promega) with luminescence as a readout. The ATP read is normalized to the control groups of every run, ruling out the variation in ATP reading due to instrumentation or basal ATP level of different donors.

### 2. Small molecule sorption in drug development and preclinical screening

Toxicity testing of several drugs in 3D primary human hepatocyte (PHH) spheroid culture (C. C. Bell et al., Drug Metab Dispos. 45, 419-429 (2017)) is performed. 3D PHH spheroid culture exhibits the highest sensitivity and selectivity by far as an in-vitro platform to predict hepatotoxicity (S. U. Vorrink et al., Toxicol. Sci. 163, 655-665 (2018) and Y. Zhou et al., Front. Pharmacol. 10 (2019), p. 1093), making it an ideal model. Since OSTE offers some advantages as a culture substrate over commonly used PTFE, including the transparency of the polymers, OSTE and PDMS are selected as low- and high-binding substrates, respectively. For the drugs, TAM, CPZ, APAP, and MON are selected as TAM and CPZ show significantly different sorption in both polymers while APAP and MON do not. Notably, CPZ shows a steep dose-response curve in 3D PHH grown in conventional ultra-low attachment (ULA) plates (C. C. Bell et al., Drug Metab Dispos. 45, 419-429 (2017)), making it an ideal test compound to accentuate the effect of differential drug.

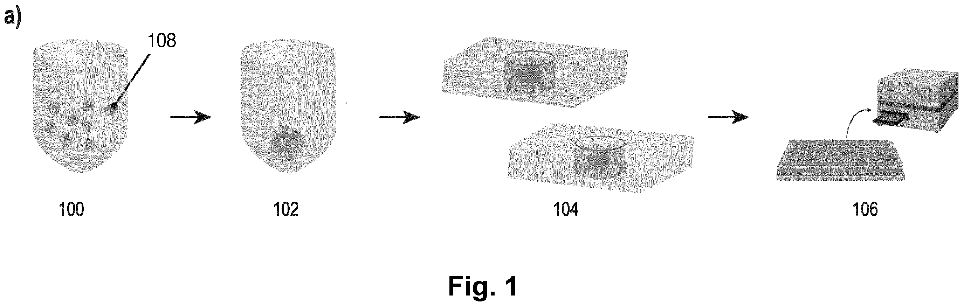

Cryopreserved PHH 108 is seeded in ULA plates 100 and once the spheroids 102 formed, isogenic PHH microtissues are transferred to either PDMS or OSTE devices 104 and exposed to several doses of respective drugs 106 (Fig. 1a).

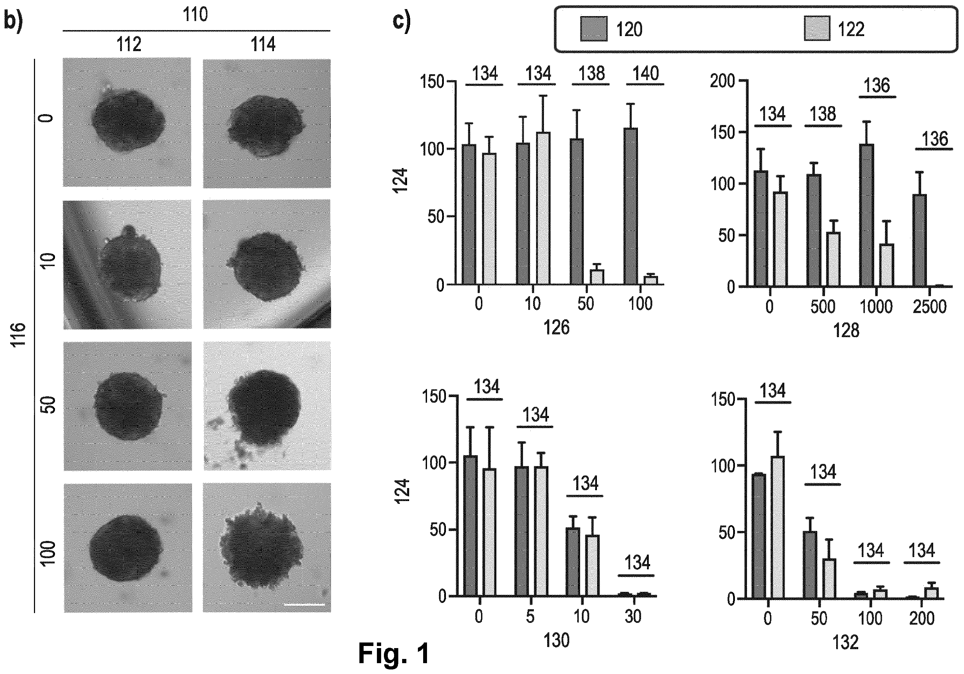

PHH spheroids maintained in PDMS remained viable in all doses of CPZ tested, in a stark contrast with those maintained in OSTE in which the spheroids exhibited loss of cellular integrity at the high doses. Fig. 1b shows in this regard CPZ concentration (µm) 116 for materials with different sorbtion 110, PDMS 112 and OSTE 114.

Using ATP assay to measure cell viability, it has been found that the toxicity of CPZ is grossly underestimated when cultured in the high-sorbing polymer devices, particularly PDMS, in which the cells remained viable even at the highest concentration tested (115.1 ± 60% vs. 5.9 ± 6.4% viability in PDMS vs. OSTE, respectively. ****P<0.0001, unpaired two-sided Student's t test, n = 11 biological replicates).

Similarly, toxicity of TAM is significantly underestimated in PDMS (89.4 ± 49.4 vs. 1.0 ± 0.2% viability in PDMS vs. OSTE, respectively, for PHH exposed to 2.5 mM TAM. *P=0.01, unpaired two-sided Student's t test n = 5-7 biological replicates). On the other hand, both APAP and MON show a similar dose-response profile. Fig. 1c shows viability (%) 124 of the cells for different concentrations of CPZ (µM) 126, TAM (µM) 128, APAP (mM) 130, and MON (µM)132, respectively, in polymer devices made from PDMS 120 or OSTE 122. Bars represent mean ± s.e.m. (n = 9-11 biological replicates for CPZ 126, 5-8 biological replicates for TAM 128, and 4 biological replicates for APAP 130 and MON 132). Two-sided unpaired Student's t test. n.s. (134), *P< 0.05 (136), **P<0.01 (138), and ***P<0.001 (140).

In the low-sorbing polymer OSTE, the half-maximal inhibitory concentration (IC₅₀) of CPZ is between 10-50µM after 24h exposure. Taking into account the sorption rate of CPZ on an OSTE material (about 70%), the IC₅₀ falls in between 3-15µM, which agrees with the reported toxic plasma dose in human (i.e., 1.5-6µM) (M. Schulz et al., Crit. Care 2020 241. 24, 1-4 (2020)). A good concordance with human toxicity data is also seen for MON and APAP. IC₅₀ of MON fell between 10-50µM, which equals to 0.4-2µM after considering the sorption rate (about 96%), in a good agreement with reported toxic dose of ~1µM (M. Schulz et al., Crit. Care 2020 241. 24, 1-4 (2020)). In both polymers, APAP exposure exhibits toxicity only at concentration of 5mM or higher, which is somewhat higher than acute toxic dose reported in human, i.e., 2-4mM (M. Schulz et al., Crit. Care 2020 241. 24, 1-4 (2020)). Notably, TAM toxic dose in an acute setting has not been reported, while exposing PHH microtissues to TAM in low-sorbing devices, i.e., OSTE, shows that the IC₅₀ of TAM is ~75µM after 24h exposure (500µM nominal dose with about 85% sorption).

Overall, these data indicate that the discrepancy in toxicity testing outcome is due to differential drug sorption on the polymers. In addition, a good concordance with human toxicity data is achieved after taking into consideration the sorption profile of culture devices used to maintain the primary human hepatocyte microtissues.

The toxicity assay results demonstrate that high-sorbing materials severely underestimate the toxicity of drug with a medium polarity, particularly TAM (logP of 6.35) and CPZ (logP of 4.54) exhibiting significantly different sorption in both devices. On the other hand, there is no significant difference in dose-response profile for APAP (logP of 0.91) and MON (logP of 8.49).

### 3. Effect of coatings

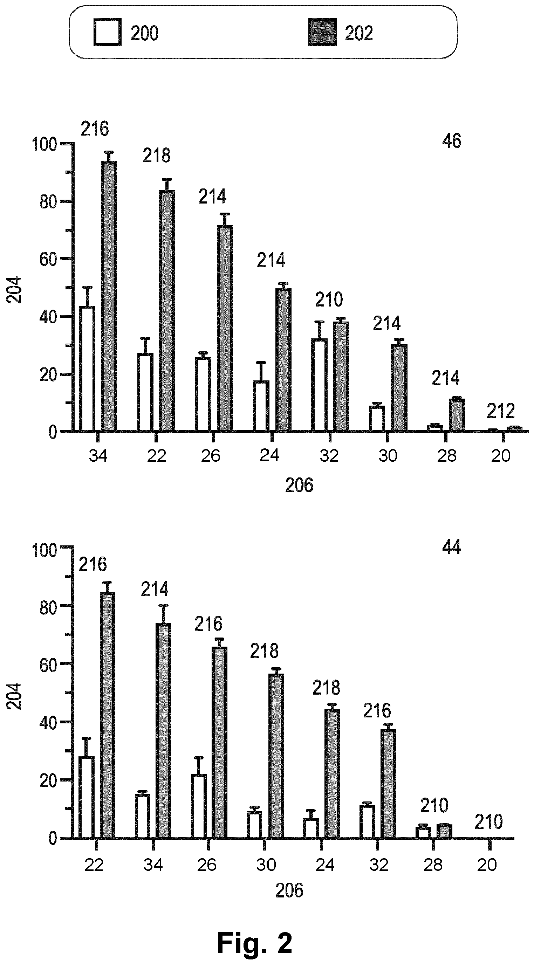

To examine whether a coating possibly improves the drug sorption profile, a drug sorption experiment with polymer devices with or without a Biofloat coating (faCellitate, Germany) is performed. Prior to use, each polymer device is coated with Biofloat for 3 min., and then air-dried for 30 min at room temperature, according to the manufacurer's instructions. TAM and CPZ are selected as they show extreme sorption in the polymers tested. Notably, the highest drug recovery is observed in OSTE and PTFE. After 24h, the mean free concentration of CPZ in OSTE and PTFE increased from 44 to 94% and from 27 to 84%, respectively. Fig. 2 shows drug recovery (%) 204 for uncoated 200 and coated 202 polymer devices made from PDMS 20, PTFE 22, COC 24, PP 26, PS 28, PC 30, PMMA 32, and OSTE 34, respectively, provided with CPZ (46) and TAM (44). Bars represent mean ± s.e.m (n = 6 independent replicates for uncoated devices, and 3 for coated devices. Two-sided unpaired Student's t test. n.s. (210) *P< 0.05 (212), **P<0.01 (214), ***P<0.001 (216), and ****P<0.0001 (218).

Similarly, on average, 84% and 74% of TAM is recovered from the solution after 24h incubation in PTFE and OSTE devices, respectively. This improvement does not apply to PDMS devices, wherein >99.9% of TAM and >97.5% of CPZ partitioned into the polymer bulks even with the coating. PS, the common material for tissue culture plates, exhibits low CPZ recovery (<20%) with the coating (although significant) compared to uncoated PS. This implicates that selection of the polymer substrata with appropriate coating may assist with difficulties associated with sorption of some drugs, particularly TAM and CPZ, but this approach may not work for some polymers, such as PDMS and PS.

## Claims

1. Polymer Device for in-vitro drug evaluation, **characterized in that** the device is comprised of a main body comprising a thiol-ene-based polymer.

2. The polymer device of claim 1, wherein the device is selected from a microfluidic chip, an organ-on-a-chip, a cell-culture-device, and a multiwell plate.

3. The polymer device of claim 1 or claim 2, wherein the device is provided with a cell culture seeded on the device and with at least one drug contacting the cell culture.

4. The polymer device of claim 3, wherein the at least one drug is selected from small molecule compounds, antibodies, antiviral compounds, antibiotics, and bioactive compounds.

5. The polymer device of claim 3 or claim 4, wherein the at least one drug includes a drug having a logP of about 3.0 to about 7.5.

6. The polymer device of any of claims 1 to 5, comprising a layer coated on the main body.

7. Use of a polymer device comprising a thiol-ene based polymer main body for in-vitro drug evaluation.

8. The use of claim 7, wherein the in-vitro drug evaluation is in-vitro drug toxicity evaluation and/or in-vitro drug efficacy evaluation.

9. The use of claim 7 or 8, wherein the in-vitro drug evaluation is performed with a cell culture seeded on the device and with at least one drug.

10. Method for assaying the toxicity and/or efficacy of at least one drug on a cell culture, the method comprising the steps of
a. providing a polymer device comprised of a main body comprising a thiol-ene based polymer;
b. seeding of a cell culture on the polymer device;
c. contacting, over a certain time period, the cell culture on the polymer device with a certain amount of the at least one drug to be assayed; and
d. assaying characteristics of the cell culture after the time period of contacting with the at least one drug and comparing the assayed characteristics to the characteristics of the cell culture prior to the time period of contacting with the at least one drug, wherein the characteristics allow conclusions on the toxicity and/or the efficacy of the at least one drug on the cell culture.
